Europäisches Patentamt

European Patent Office

Office européen des brevets

(11) Numéro de publication: **0 112 203**
**B1**

(12) # FASCICULE DE BREVET EUROPÉEN

(45) Date de publication du fascicule du brevet:
03.09.86

(51) Int. Cl.⁴: **G 03 C 5/16,** G 02 F 1/33,
G 01 T 1/29, A 61 B 6/02

(21) Numéro de dépôt: **83402191.7**

(22) Date de dépôt: **10.11.83**

(54) **Appareil d'imagerie médicale permettant la tomographie longitudinale.**

(30) Priorité: **17.11.82 FR 8219228**

(43) Date de publication de la demande:
**27.06.84 Bulletin 84/26**

(45) Mention de la délivrance du brevet:
**03.09.86 Bulletin 86/36**

(84) Etats contractants désignés:
**DE GB NL**

(56) Documents cité:
**EP-A-0 031 952
EP-A-0 077 678
DE-A-2 719 068
FR-A-2 514 908
US-A-3 617 931
US-A-4 114 987
US-A-4 179 100
US-A-4 239 968**

(73) Titulaire: **COMMISSARIAT A L'ENERGIE
ATOMIQUE Etablissement de Caractère
Scientifique Technique et Industriel, 31/33, rue
de la Fédération, F-75015 Paris (FR)**

(72) Inventeur: **Allemand, Robert, 17, Chemin de
Bouffière, F-38330 Saint Ismier (FR)**
Inventeur: **Cuzin, Marc, "Les Cèdres" 24, Rue
Jean Jaurès, F-38610 Gieres (FR)**
Inventeur: **Parot, Pierre, "Le Colombier", F-04100
Manosque (FR)**

(74) Mandataire: **Mongrédien, André, c/o BREVATOME
25, rue de Ponthieu, F-75008 Paris (FR)**

EP 0 112 203 B1

LIBER, STOCKHOLM 1986

## Description

La présente invention a pour objet un appareil d'imagerie médicale permettant notamment la tomographie longitudinale.

Un écran radiuminescent stimulable exposé à un rayonnement comme des rayons X, des rayons $\alpha$, des rayons $\beta$ ou des rayons $\gamma$ emmagasine une partie de l'énergie du rayonnement reçu. L'image de rayonnement est donc mémorisée dans l'écran radioluminescent. Ensuite, lorsque cet écran radioluminescent stimulable est exposé à un rayonnement de stimulation, il émet un rayonnement lumineux en fonction de l'énergie de rayonnement emmagasinée. Ce rayonnement lumineux est capté par un photodétecteur qui le transforme en un signal électrique exploitable.

On connaît de tels dispositifs de lecture d'une image de rayonnement contenue dans un écran radioluminescent stimulable, notamment par le brevet américain n° 4 239 968 du 16 décembre 1980. La stimulation de l'écran radioluminescent stimulable requiert un rayonnement de stimulation de forte énergie, c'est pourquoi la stimulation se fait en général par un faisceau laser stimulant un point de l'écran radioluminescent, ce faisceau de rayons de stimulation étant dévié par un moyen de déflexion de manière à balayer point par point tout l'écran radioluminescent stimulable.

Les moyens de déflexion utilisés dans les dispositifs de lecture d'une image de rayonnement selon l'art connu sont le plus souvent des miroirs tournants. Il s'agit donc d'un moyen de déflexion comprenant des pièces mobiles, ce qui limite la vitesse de balayage de l'écran radioluminescent stimulable. Cette vitesse de balayage est déterminée par le temps d'accès aléatoire qui mesure la durée nécessaire au faisceau de rayons de stimulation pour passer d'un point de l'écran radioluminescent stimulable à un autre point quelconque de l'écran radioluminescent stimulable. Ce temps d'accès aléatoire est de l'ordre de 1 ms pour un faisceau de rayons de stimulation dévié par un miroir tournant. Le temps de stimulation de la totalité de l'écran radioluminescent stimulable est alors de l'ordre de la minute. En radiologie medicale ce temps de lecture n'est pas une contrainte car on a le temps de lire l'écran radioluminescent stimulable entre deux radiographies. Par contre, ce temps de lecture de l'écran radiolumimescent stimulable empêche de faire de la tomographie longitudinale où il faut prendre plusieurs radiographies rapidement, c'est-à-dire à des intervalles de l'ordre de la seconde.

La présente invention a pour but d'améliorer le temps d'accès aléatoire à un point de l'écran radioluminescent stimulable en vue de faire, notamment, de la tomographie longitudinale.

La présente invention consiste notamment à doter les dispositifs de lecture d'images de rayonnement d'un moyen de déflexion ne comportant pas de pièces mobiles, ce qui assure une plus grande vitesse de déflexion et une meilleure précision, donc un temps d'acces aléatoire moindre.

L'invention a pour objet un appareil d'imagerie médicale permettant la tomographie longitudinale comprenant:
- une surface de réception d'un patient
- une source de rayonnement X ou $\gamma$, mobile selon une direction parallèle à la surface et munie de collimateurs mobiles selon un axe parallèle à ladite surface et perpendiculaire à la direction de déplacement de la source,
- un écran radioluminescent stimulable, mobile selon une direction parallèle à celle de la source de rayonnement et interceptant le faisceau de rayonnement ayant traversé le patient,
- un dispositif de lecture émettant un faisceau de stimulation pour lire un écran radioluminescent stimulable contenant une image de rayonnement,
- un photodétecteur pour recevoir l'image lumineuse émise par l'écran radioluminescent stimulé par le faisceau de stimulation,
- des moyens de traitement du signal électrique délivré par le photodétecteur
ledit appareil étant caractérisé en ce qu'il comprend un ensemble d'écrans radioluminescents stimulables et en ce que le dispositif de lecture comprend un moyen de déflexion de type acousto-optique pour balayer le faisceau de stimulation sur la surface d'un écran radioluminescent stimulable.

Selon une autre caractéristique, l'écran radioluminescent stimulable est continu.

L'invention sera mieux comprise à la lecture de la description qui suit d'un exemple de réalisation donné à titre indicatif et non limitatif, en référence aux dessins annexés sur lesquels:
- la figure 1 représente, vu de dessus, un dessin schématique d'un dispositif de lecture conforme à l'invention;
- la figure 2a represente schématiquement, vu de face, un appareil de tomographie longitudinale utilisant le dispositif de lecture selon l'invention;
- la figure 2b représente le même appareil de tomographie vu de côté;
- les figures 3 et 4 représentent différents systèmes de mise en place d'écrans radioluminescents stimulables.

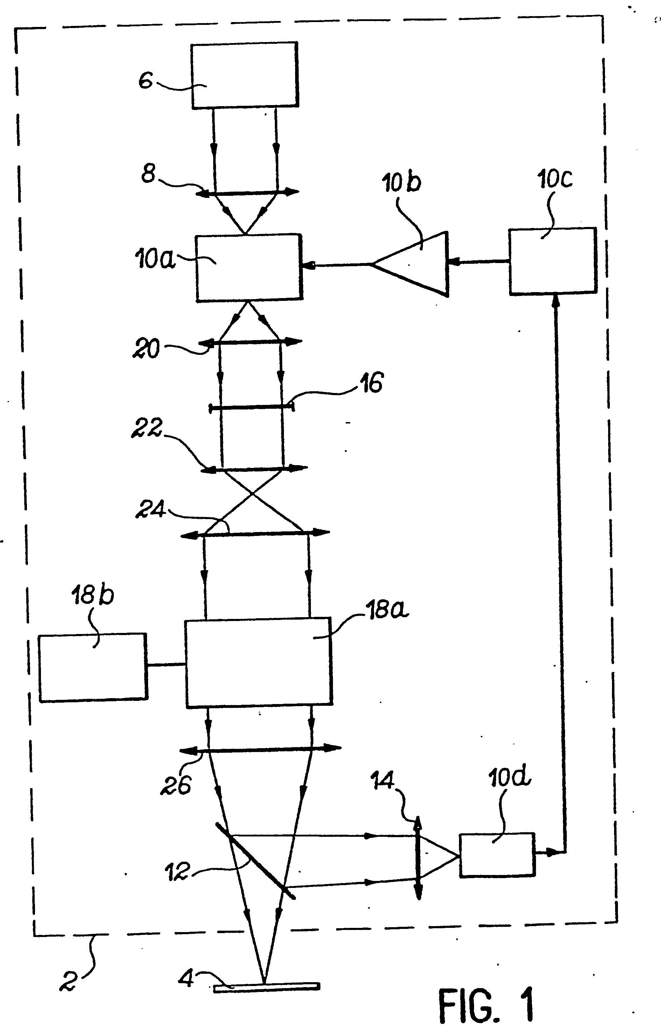

Sur la figure 1, on a représenté un dispositif de lecture 2 d'une image de rayonnement contenue dans un écran radioluminescent 4 stimulable. Pour balayer l'écran radioluminescent 4 stimulable, il faut deux moyens de déflexion, l'un réalisant une déflexion horizontale du faisceau de rayonnement de stimulation et l'autre une déflexion verticale de ce faisceau. Pour simplifier le schéma de la figure 1, on a supposé que l'écran radioluminescent 4 stimulable était linéaire; un seul moyen de déflexion est alors nécessaire.

Le dispositif de lecture 2 comprend une source 6 générant le faisceau de rayons de stimulation. Cette source 6 peut être un laser, par exemple un laser He-Ne, un laser à rubis, un laser à krypton ou autre. Cette source 6 sera choisie telle que la longueur d'onde de son rayonnement soit suffisamment différente de la longueur d'onde du rayonnement lumineux émis par l'écran radioluminescent 4 stimulable pour que le photorécepteur soit sensible au second rayonnement et non au premier. La mesure du rayonnement émis peut être facilitée par un filtrage adéquat.

Le faisceau de rayons de stimulation issu de la source 6 est focalisé par une lentille 8 sur un moyen de contrôle 10 de l'intensité de la source 6. En effet, le rayonnement lumineux émis par l'écran radioluminescent 4 stimulable varie avec l'intensité du faisceau de rayons de stimulation. Il importe donc de stimuler l'écran radioluminescent 4 stimulable avec un faisceau de rayons de stimulation d'intensité constante pour avoir une image de rayonnement lumineux identique à l'image mémoire dans l'écran radioluminescent 4 stimulable.

Ce contrôle est effectué par un détecteur 10d recevant une partie du faisceau de rayons de stimulation réfléchi par une lame semi-transparente 12 et focalisé par une lentille 14. Ce détecteur 10d est relié à un oscillateur 10c qui commande au travers d'un amplificateur 10b un modulateur 10a, par exemple de type acousto-optique. Cette boucle de contrôle du faisceau de rayons de stimulation assure une stimulation uniforme de l'écran radioluminescent 4 stimulable. Le faisceau de rayons de stimulation sortant du modulateur 10a traverse un polariseur 16 qui oriente le plan de polarisation de l'onde de stimulation dans une direction convenable pour le déflecteur 18a. Des lentilles 20, 22 et 24 sont placées entre le modulateur 10a et le déflecteur 18a; leur rôle de focalisation et de défocalisation apparaît clairement sur la figure 1. Le moyen de déflexion 18, de type acousto-optique, comprend le déflecteur 18a et un moyen de commande 18b. Le moyen de commande 18b génère un signal électrique de fréquence de l'ordre de 100 à 200 MHz, d'énergie quelques watts. Ce signal électrique appliqué sur un cristal piézoélectrique transforme cette onde électrique en une onde de pression qui traverse le déflecteur 18a, constitué par exemple d'un cristal tel que $MoPbO_4$, faisant varier son indice optique n et par conséquent déviant le faisceau de rayons de stimulation qui est ensuite focalisé par la lentille 26 sur l'écran radioluminescent 4 stimulable. Le signal électrique est bien entendu ajusté de façon que le faisceau de rayons de stimulation balaie tout l'écran radioluminescent 4 stimulable.

Un tel dispositif de lecture, muni d'un moyen de déflexion acousto-optique, a un temps d'accès aléatoire de 10 microsecondes ou moins, 100 fois moindre que celui d'un dispositif de lecture muni d'un moyen de déflexion par miroir tournant. Ce gain de temps de lecture permet de faire de la tomographie longitudinale. Mais il peut aussi se révéler intéressant pour la lecture rapide de toute image de rayonnement.

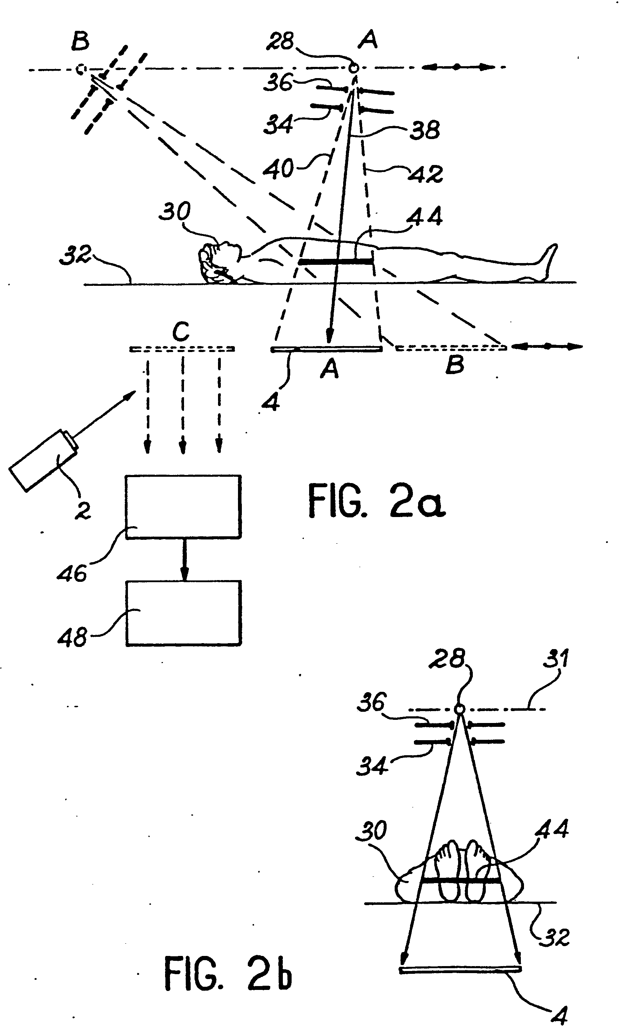

Les figures 2a et 2b représentent schématiquement un appareil de tomographie utilisant le dispositif selon l'invention. Cet appareil comprend une source de rayonnement 28 traversant un patient 30 placé sur une surface plane 32. Le rayonnement est reçu par l'écran radioluminescent 4 stimulable situé sous la surface plane 32. Un collimateur 34 limite la largeur du faisceau de rayonnment à la largeur de l'écran radioluminescent 4 stimulable, le terme de largeur s'appliquant aux dimensions transverses. Un collimateur 36 limite l'épaisseur du faisceau de rayonnement 38 à un trait. Ces deux collimateurs 34 et 36 sont mobiles autour d'un axe 31, visible sur la figure 2b. Par déplacement de ce collimateur 36, le faisceau de rayonnement peut se déplacer entre les limites extrêmes 40 et 42 pour balayer la longueur de l'écran radioluminescent 4 stimulable. L'appareil de tomographie comprend en outre un dispositif de lecture 2 conforme à l'invention, un photodétecteur 46 et des moyens de traitement 48. Par moyen de traitement, nous entendons tout moyen que l'homme de l'art utilise habituellement dans un tel appareil à savoir amplificateur, convertisseur analogique-numérique, mémoire, visualisation, etc...

La tomographie longitudinale consiste à prendre des images d'un même plan sous différents angles. La coupe longitudinale est alors reconstituée par traitement numérique et combinaison des différentes images.

La saisie d'images radiographiques pour reconstituer la coupe longitudinale 44 s'opère de la façon suivante. La source de rayonnement 28 et les deux collimateurs 34 et 36, mobiles solidairement dans le sens longitudinal comme indiqué par les flèches, sont placés dans la position, par exemple A, permettant de faire une radiographie du plan 44 sous l'angle choisi. L'écran radioluminescent 4 stimulable, mobile dans le sens logitudinal indépendamment de la source de rayonnement 28 est positionné de manière à recevoir le rayonnement issu de la source de rayonnement 28. Lorsque l'image de rayonnement est mémorisée dans l'écran radioluminescent 4 stimulable, celui-ci est déplacé en position C où il est stimulé par le dispositif de lecture 2. Le rayonnement lumineux émis capté par le photodétecteur 46 est ensuite traité par le moyen de traitement 48. La source de rayonnement 28 peut être ensuite déplacée dans une autre position telle que B et l'écran radioluminescent 4 stimulable placé en regard dans la position B correspondante pour opérer la saisie d'une autre image.

Le temps d'attente entre deux radiographies est alors au moins égal au temps de lecture de l'écran radioluminescent 4 en position C et aux temps de transfert de A en C puis de C en B. Ce temps peut être réduit si l'on dispose de plusieurs écrans radioluminescents tels que 4 stimulables, on peut alors simultanément lire un écran radioluminescent en position C et saisir une image de rayonnement sur un écran radioluminescent en position A.

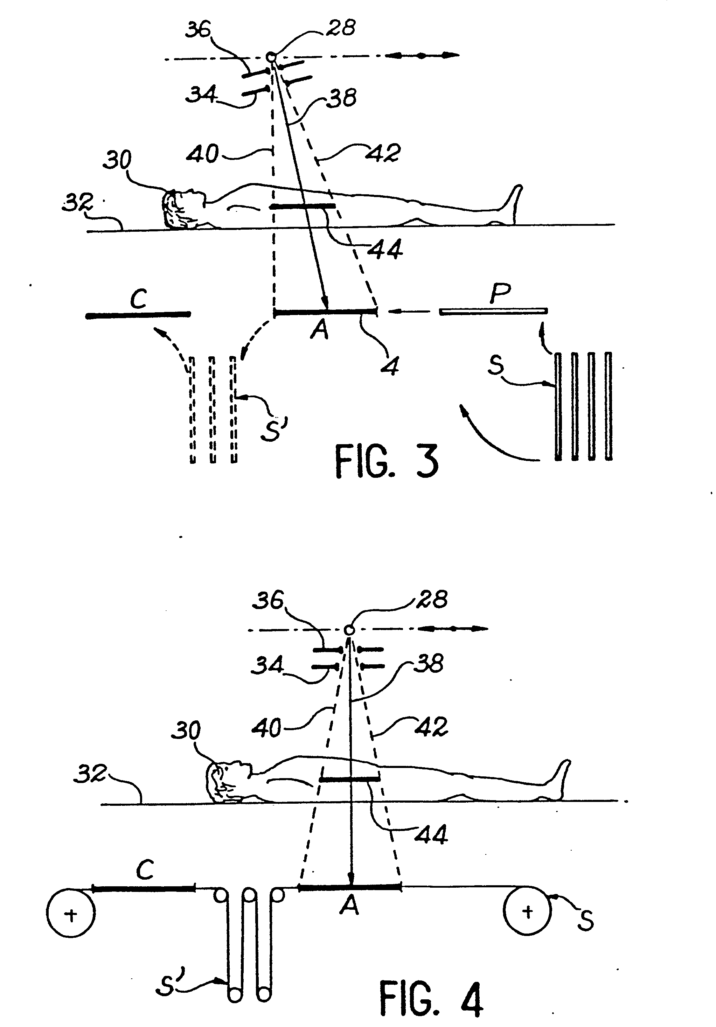

Les figures 3 et 4 donnent différentes possibilités de tels écrans radioluminescents.

La figure 3 représente un ensemble d'écrans radioluminescents tel que 4 stimulables en position de stockage S. On peut effectuer simultanément 3 opérations: lecture d'un écran radioluminescent en position C, acquisition d'image de rayonnement dans une position telle que A, préparation d'un écran radioluminescent en position P pour la prochaine radiographie. On peut aussi imaginer une position de stockage S' qui reçoit les écrans radioluminescents ayant acquis une image de rayonnement, la lecture se faisant en différé au moment le plus opportun.

La figure 4 montre une variante où les écrans radioluminescents sont remplacés par une bande radioluminescente continue à défilement séquentiel. Cette bande peut être refermée sur elle-même formant ainsi une boucle. La bobine primaire S sert de stockage d'écrans radioluminescents vierges. Entre la position d'acquisition d'image de rayonnement telle que A et la position de lecture C, on peut avoir une position de stockage S' permettant une lecture différée.

## Revendications

1. Appareil d'imagerie médicale permettant la tomographie longitudinale comprenant:
- une surface (32) de réception d'un patient (30),
- une source (28) de rayonnement X ou γ, mobile selon une direction parallèle à la surface (32) et munie de collimateurs (34, 36) mobiles selon un axe (31) parallèle à ladite surface (32) et perpendiculaire à la direction de déplacement de la source (28),
- un écran radioluminescent (4) stimulable, mobile selon une direction parallèle à celle de la source (28) de rayonnement et interceptant le faisceau de rayonnement ayant traversé le patient (30),
- un dispositif de lecture (2) émettant un faisceau de stimulation pour lire un écran radioluminescent stimulable contenant une image de rayonnement,
- un photodétecteur (46) pour recevoir l'image lumineuse émise par l'écran radioluminescent stimulé par le faisceau de stimulation,
- des moyens de traitement (48) du signal électrique délivré par le photodétecteur (46),
ledit appareil étant caractérisé en ce qu'il comprend un ensemble d'écrans radioluminescents (4) stimulables et en ce que le dispositif de lecture comprend un moyen de déflexion (18) de type acousto-optique pour balayer le faisceau de stimulation sur la surface d'un écran radioluminescent stimulable.

2. Appareil selon la revendication 1, caractérisé en ce que l'ensemble d'écrans est constitué d'une pluralité d'écrans radioluminescents distincts.

3. Appareil selon la revendication 1, caractérisé en ce que l'ensemble d'écrans est constitué par une bande radioluminescente stimulable continue.

4. Appareil selon la revendication 3, caractérisé en ce que ladite bande forme une boucle fermée.

5. Appareil selon la revendication 1, caractérisé en ce que l'ensemble d'écrans est agencé de sorte que simultanément une image de rayonnement est saisie et une autre image de rayonnement est lue.

## Patentansprüche

1. Medizinisches Sichtgerät für lineare Tomographie, enthaltend:
- eine Aufnahmefläche (32) für einen Patienten (30),
- eine Röntgen- oder γ-Strahlenquelle (28), die parallel zu der Fläche (32) beweglich ist und die mit Kollimatoren (34, 36) versehen ist, die längs einer Achse (31) beweglich sind, die zur genannten Fläche (32) parallel und zur Verschiebungsrichtung der Quelle (28) senkrecht ist,
- einen erregbaren Radiolumineszenzschirm (4), der in einer Richtung parallel zu jener der Strahlenquelle (28) beweglich ist und das Strahlenbündel aufnimmt, das den Patienten (30) durchquert hat,
- eine Ableseeinrichtung (2), die einen Erregerstrahl abgibt, um einen erregbaren Radiolumineszenzschirm, der ein Strahlungsbild enthält, abzulesen,
- einen Photodetektor (48) zum Aufnehmen des von dem durch den Erregerstrahl erregten Radiolumineszenzschirm abgegebenen Lumineszenzbildes,
- Behandlungseinrichtungen (48) für das von dem Photodetektor (46) gelieferte elektrische Signal,
wobei das Gerät dadurch gekennzeichnet ist, daß es eine Gruppe von erregbaren Radiolumineszenzschirmen (4) enthält und daß die Lesevorrichtung eine Ablenkeinrichtung (18) akustooptischer Art enthält, um den Erregerstrahl auf der Oberfläche eines erregbaren Radiolumineszenzschirms abzulenken.

2. Gerät nach Anspruch 1, dadurch gekennzeichnet, daß die Schirmgruppe von einer Mehrzahl einzelner Radiolumineszenzschirme gebildet wird.

3. Gerät nach Anspruch 1, dadurch gekennzeichnet, daß die Schirmgruppe von einem durchgehenden erregbaren Radiolumineszenzband gebildet ist.

4. Gerät nach Anspruch 3, dadurch gekennzeichnet, daß das Band eine geschlossene Schleife bildet.

5. Gerät nach Anspruch 1, dadurch gekennzeichnet, daß die Schirmgruppe so betrieben ist, daß simultan ein Strahlenbild aufgenommen und ein anderes Strahlenbild gelesen wird.

## Claims

1. Medical imaging apparatus for longitudinal tomography comprising:
   - a surface (32) for supporting a patient (30),
   - an X-radiation or γ-radiation souroe (28), moveabie in adirection parallel to the surface (32) and having collimators (34, 36) moveable along an axis (31) parallel to said surface (32) and perpendicular to the direction of displacement of the source (28),
   - an excitable radioluminescent screen (4) moveable in a direction parallel to that of the radiation source (28) and adapted to intercept a radiation beam after passage through the patient,
   - a reading device (2) emitting an excitation beam for reading an excitable radioluminesoent screen bearing a radiation image,
   - a photodetector (46) for receiving a light image emitted by the radioluminescent screen on excitation by the excitation beam, and
   - processing means (48) for an electrical signal delivered by the photodetector (46),
   said apparatus being characterised in that it comprises an assembly of excitable radioluminescent screens (4) and in that the reading device comprises an acoustic-optical deflector (18) for causing the excitation beam to scan the surface of an excitable radioluminescent screen.

2. Apparatus according to claim 1, characterised in that the screen assembly comprises a plurality of separate radioluminescent screens.

3. Apparatus according to claim 1, characterised in that the screen assembly comprises an excitable radioluminescent continuous belt.

4. Apparatus according to claim 3, characterised in that said belt forms a closed loop.

5. Apparatus according to claim 1, characterised in that the screen assembly is actuated such that a radiation image is acquired simulaneously with reading of another radiation image.

FIG. 1

FIG. 2a

FIG. 2b

FIG. 3

FIG. 4